# NEW EUROPEAN PATENT SPECIFICATION

(11) **EP 2 091 503 B2**
(45) Date of publication and mention of the opposition decision: **27.05.2015**
(45) Mention of the grant of the patent: 16.03.2011
(21) Application number: 07847254.5
(22) Date of filing: 21.11.2007
(51) Int. Cl.: A61K 8/26, A61K 8/28, A61K 8/31, A61Q 15/00, A61K 8/89, A61K 8/02, A61K 8/06

(54) **ANTIPERSPIRANT STICK COMPOSITIONS**
ANTIPERSPIRANSSTIFT ZUSAMMENSETZUNGEN
COMPOSITIONS POUR BÂTONNET ANTI-TRANSPIRATION

(30) Priority: 20.12.2006 US 870878 P
(43) Date of publication of application: 26.08.2009
(73) Proprietor: Unilever PLC, London EC4Y 0DY (GB); Unilever N.V., 3013 AL Rotterdam (NL)
(72) Inventor: CROPPER, Martin Peter, Bebington, Wirral Merseyside CH63 3JW (GB); BIANCHI, James Michael, Trumbull, Connecticut 06611 (US); EMSLIE, Bruce Steven, Leeds, Yorkshire LS14 2AR (GB); FRANKLIN, Kevin Ronald, Bebington, Wirral Merseyside CH63 3JW (GB); ROBERTS, Louise Jannette, Bebington, Wirral Merseyside CH63 3JW (GB); STOCKTON, Joanne Elizabeth, Bebington, Wirral Merseyside CH63 3JW (GB)
(74) Representative: McHugh, Paul Edward
(86) International application number: PCT/EP2007/062625
(87) International publication number: WO 2008/074586

(56) References cited:
- EP-A- 1 374 843
- WO-A-01/51020
- WO-A-99/51192
- WO-A-2004/089325
- US-A1- 2005 281 767
- US-A1- 2005 287 069

## Description

The present invention relates to astringent stick compositions, especially to such compositions containing an astringent antiperspirant salt and in particular to compositions in the form of solidified water in oil emulsions

Compositions containing an astringent antiperspirant salt act as deodorants when applied topically to skin, inhibiting the generation in situ of malodorous compounds from solutes in aqueous excretions from eccrine and/or apocrine glands, by virtue of their bactericidal properties, even when the astringent salt is present in only a low concentration which in practice would be deemed sufficient to be classified as an antiperspirant composition. As the proportion of the astringent antiperspirant salts increases, so the composition becomes increasingly capable to act additionally as an antiperspirant, commonly by blocking the pore of eccrine and/or apocrine glands.

Compositions containing an astringent antiperspirant salt, conventionally, can be classified according to their physical characteristics, including particulate mixtures and liquid compositions, semi-solid or cream compositions and solid stick compositions. Sticks are characterised by being integral and self-supporting, commonly in the form of a rod or bar, although for ease of handling they are usually dispensed from a tubular container having an open end and provided with a platform or piston that is moveable towards the open end and a removable cap. Some compositions containing an antiperspirant salt are anhydrous, by which is usually meant that the composition contains no separate aqueous phase, and others are aqueous compositions in the form of either solutions or emulsions. In solutions, conventionally, there is no additional non-aqueous liquid phase, and in emulsions there is conventionally an oil phase in addition to the aqueous phase. In emulsions, the antiperspirant active is commonly dissolved in the aqueous phase forming an aqueous acidic solution.

The instant invention relates in particular to astringent stick formulations which comprise droplets of a solution of an antiperspirant active dispersed in a solidified continuous oil phase.

Particular difficulties are presented in the manufacture of astringent stick compositions that are in the form of water in oil emulsions having desirable properties. By way of example, a high, if not overwhelming, proportion of commercially available antiperspirant sticks are anhydrous and comprise an oil phase, and typically these days contain a volatile silicone oil, in which a particulate antiperspirant active is suspended, which oil is solidified by a major fraction of a linear fatty alcohol such as stearyl alcohol supplemented by a minor fraction of a plant-derived wax such as castor wax. Regrettably, it is not possible simply to transfer stick-making technology from an anhydrous stick to a water-in-oil emulsion stick. The presence of the dispersed aqueous phase can result in an integral solid mass not being formed or, if a solid stick is formed, it can subsequently fall apart even under very mild pressure and/or at elevated storage temperatures that can occur during the summer, such as in southern States of the USA. Thus, even if the same solidifying agent, such as a wax, has been disclosed as being suitable to make an anhydrous antiperspirant stick, there can be no certainty that it could be employed successfully to make an emulsion stick, and, indeed, a significant risk for a wax that it would fail. Moreover, there can be no certainty that even if a structurant could successfully make a stick, that the resultant stick would exhibit a desirable combination of sensory and/or visual attributes.

The problem of emulsion stick formation has been addressed in a series of patents to Unilever, including US 6287544, US 6455056, US 6248312 and WO 2003/059307. These specifications disclose that emulsion sticks can be made using structurants that meet certain defined characteristics, many of which structurants are fibre-forming small molecule gelling agents. A combination of structurants has been disclosed in WO 2004/098551, also to Unilever, that likewise can make emulsion sticks under specified conditions. Regrettably, many of the structurants described are not available commercially so that their implementation would involve a substantial programme of work to first develop an acceptable commercial manufacture process and also to satisfy regulatory authorities in countries constituting major markets for antiperspirant sticks, including in particular the USA and also the EU. Such procedures are time-consuming and costly. Moreover, manufacturing temperatures employing such structurants are commonly higher than when employing conventional wax structurants which introduces additional processing costs and reduces plant flexibility. Accordingly, it is desirable to seek an alternative solution to the problem and in particular one that enables a wax to be employed.

In US 6387358 to Unilever, there is described, in one set of Examples, antiperspirant soft solid compositions in which an oil phase is thickened (not solidified) by a polyethylene wax, by itself or with a further thickener, but this provides no teaching to the use of such material to form an emulsion stick, because the exemplified formulations therein are not only not in the form of a firm stick, but importantly are anhydrous. Emulsions stick formulations suffer from different problems from anhydrous stick formulations, arising, for example, from the absence or presence of an aqueous phase.

Whilst it is a pre-requisite that, in practice, a stick formulation can not only be made but also does not disintegrate before use by the consumer, the aesthetics of the stick are of considerable significance to a consumer as well, and can make an important contribution as to whether the consumer purchases the product again. Amongst other attributes that a stick user takes into account is the perceptions of the stick as it is wiped across the skin surface (viz. drag) and of skin on skin contact (viz. glide). There is commonly a preference for products having lower rather than higher drag and for products which glide more easily across skin compared with those which do not. It will be recognised that the attributes of a product when applied using the same dispenser, including in particular stick products, derive significantly from the combination of all the ingredients employed in the formulation.

It will also be recognised that it is inherently preferable for a stick to attain a desirable hardness. A stick that is softer can result in increased deposition, which can be wasteful and/or a sensation of stickiness which is not pleasant. However, merely expressing an aspiration to attain a desirable hardness does not teach how to attain it.

Moreover, a significant fraction of consumers of antiperspirant compositions in many countries consider an important aesthetic attribute the appearance of an antiperspirant composition on application to skin and/or whether marks are visible if the composition should transfer to clothing that comes into contact with the composition. The latter can arise from direct contact or by some of the composition being dislodged from the skin and falling onto clothing. The appearance of white marks is commonly attributed to wax structurants in antiperspirant compositions containing them, either by themselves and/or in conjunction with the astringent antiperspirant salt. As above, expressing an aspiration to improve the aesthetic attributes of a stick composition does not teach how to make such an improvement.

Antiperspirant water-in-oil emulsions have been described in EP 1280502 in which the oil phase is structured with an ester wax. Compared with anhydrous antiperspirant compositions, such emulsions have exhibited reduced visible whitening, but it would be desirable to be able to make compositions which continued to employ a wax, and which exhibited even less visible whitening.

A water-in-oil emulsion stick containing 15% by weight of potassium alum in the aqueous phase and in which the oil phase is gelled with polyethylene, namely Polywax 500, has hitherto been described in Example 1 of USP 6139824. In col. 1, lines 17 to 21, the text teaches against the use of aluminium antiperspirant salts, and instead teaches the use of alum salts of low solubility. Alum solutions when tested exhibited a number of practical disadvantageous characteristics such as the formation of small white crystals in situ during temperature cycling storage and the formulated stick formulations suffer from problems of leakage, and distinct, white marks if the composition is wiped across cloth. Accordingly, and particularly in the context of seeking to improve visible whitening, USP 6139824 does not provide any inducement to employ a polyethylene wax to structure an antiperspirant formulation.

It is an object of at least some embodiments of the present invention to create a water-in-oil antiperspirant formulation that employs a wax to solidify the oil phase and which ameliorates or overcomes one or more of the disadvantages identified hereinabove.

It is another object of some or other embodiments of the present invention to create a water-in-oil wax structured antiperspirant formulation exhibiting desirable aesthetics and preferably exhibiting a low drag and/or easy glide when wiped across a skin surface.

It is a yet other object of certain of the embodiments of the invention described herein to create a water-in-oil antiperspirant emulsion having an oil phase structured with a wax which exhibits less or no worse than low visible marks.

It is an object of various preferred embodiments of the present invention to create a water-in-oil wax structured antiperspirant emulsion which not only exhibits low drag and/or easy glide on application to skin, but also does not produce high visible marks.

### Brief summary of the present invention

According to one aspect of the present invention, there is provided an antiperspirant composition containing an astringent antiperspirant salt and structured with a hydrocarbon wax which itself comprises a polyethylene wax having an average molecular weight of from 360 to 460 Daltons, which is sometimes otherwise referred to herein as intermediate weight polyethylene.

In this aspect, the emulsion desirably comprises:- from 34% to 70% by weight of a dispersed aqueous phase containing from 15 to 30% by weight of a water-soluble astringent aluminium-zirconium antiperspirant salt from 29% to 65% by weight of an oil phase comprising at least one oil and a hydrocarbon wax that solidifies the oil, the hydrocarbon wax comprising at least 1 part w/w of polyethylene of intermediate molecular weight being a weight average molecular weight of from 360 to 460 daltons per 8 parts of oil phase and from 0.125% to 2.0% by weight of a silicone emulsifier. Herein percentages are by weight of the composition unless explicitly stated otherwise.

Said intermediate weight polyethylene wax has proven to be especially effective for creating a firm stick containing as a dispersed phase a substantial proportion of a solution of an astringent antiperspirant salt, one that typically has a substantially greater acidity than water by itself, an effectiveness to be contrasted with use of other waxes by themselves, for example other hydrocarbon waxes, silicone waxes, ester waxes, including hydrogenated ester oils, and linear fatty alcohols and also differentiates from polyethylene waxes of lower molecular weight. The effectiveness of a wax is observed by its capability to form a stick, and/or one or more of the aesthetic attributes of a stick that is formed.

By virtue of the selection of the constituents, including in particular the selection of a hydrocarbon wax containing essentially the intermediate molecular weight polyethylene, it is possible to obtain an emulsion stick that is formed using a wax as gellant and preferably a stick that exhibits low drag when wiped across human skin surface, such as for example skin in the human axilla, and/or easy glide after application.

According to a second aspect of the present invention, there is provided a process for the preparation of an antiperspirant stick in which a hydrocarbon wax comprising polyethylene having a weight average molecular weight of from 360 to 460 daltons is dissolved in an oil to form an oil phase, an aqueous solution of an antiperspirant salt dissolved in water is dispersed in the oil phase in the presence of an emulsifying agent and the oil phase is caused or permitted to solidify, the weight proportions of ingredients being in accordance with the first aspect.

According to a fifth aspect of the present invention there is provided a non-therapeutic method of locally inhibiting or controlling odour or perspiration, as the case may be, in which a stick composition according to the first aspect is wiped across human skin, and preferably in the axilla.

### Detailed Description and Preferred Embodiments.

The present invention relates to the employment of the intermediate molecular weight polyethylene as the primary and essential wax gellant in a hydrocarbon wax employed in a sufficient concentration for gelling the continuous, oil phase of an emulsion in which the dispersed phase comprises an aqueous solution of an astringent antiperspirant salt, thereby solidifying the emulsion. Primary indicates a supplementary wax can be contemplated, but that it should provide no more than half the total weight of the waxes, and preferably less. The use of the intermediate molecular weight polyethylene wax enables a firm stick to be made using an emulsion formulation, thereby retaining the benefit of the antiperspirant active being in an aqueous phase rather than in particulate form as is usual in an anhydrous stick whilst obtaining the benefit of a stick with comparable hardness to that obtainable from an anhydrous composition. By so doing, the resultant solidified emulsion also exhibits various aesthetic advantageous properties, including, in particular good resistance to drag and good glide. Various formulations alternatively or additionally exhibit commendably low visible marks, for example even by comparison with emulsion sticks made using ester waxes. Herein, reference to an average molecular weight of the polyethylene wax is to a weight average. The emulsion comprises a third constituent, namely an emulsifier, located at the interface between the continuous and dispersed phases, viz. the oil and aqueous phases.

### Dispersed Phase

The dispersed phase comprises an aqueous solution of an astringent antiperspirant salt and its combined weight with the emulsifier is usually from 34% to 70% by weight of the emulsion. The weight proportion of the dispersed phase is preferably at least 35%, in many desirable embodiments up to 60% and particularly up to 50%.

The weight concentration of the antiperspirant salt in the aqueous phase is commonly not higher than 65%, and for an antiperspirant stick is advantageously at least 35% and in many preferred embodiments its weight concentration is from 45 to 60%. The weight of antiperspirant salt based on the entire emulsion is usually at least 10% and in practice is commonly up to 30%. A preferred range of antiperspirant salt concentration for the antiperspirant stick is from 15% to 26%. Such total concentration of antiperspirant salt in the emulsion can be achieved by appropriate selection together of the proportion of aqueous phase and the concentration of salt in that phase.

### Astringent antiperspirant Salts

The astringent antiperspirant salts for use herein are selected from astringent mixed aluminium/zirconium salts, which may comprise complexes. Preferred astringent salts include halogen-containing aluminium/zirconium salts, especially chloro salts and most particularly halohydrate salts, such as preferably chlorohydrates. Such mixed salts can be considered, empirically, to consist of an aluminium salt and a zirconium salt, though in practice, the astringent material that is present in the composition consists of a number of related species, commonly polymeric, containing both aluminium and zirconium atoms in different molar proportions and having differing molecular weights.

Aluminium halohydrates are usually defined by the general formula Al₂(OH)ₓQ_{y}.wH₂O in which Q represents chlorine, bromine or iodine, x is variable from 2 to 5 and x + y = 6 while wH₂O represents a variable amount of hydration.

Zirconium astringent salts can usually be represented by the empirical general formula: ZrO(OH)_{2n-nz}B_{z}.wH₂O in which z is a variable in the range of from 0.9 to 2.0 so that the value 2n-nz is zero or positive, n is the valency of B, and B is selected from the group consisting of chloride, other halide, sulphamate, sulphate and mixtures thereof. Possible hydration to a variable extent is represented by wH20. Preferable is that B represents chloride and the variable z lies in the range from 1.5 to 1.87. In practice, herein, such zirconium salts are employed as a component of a combined aluminium and zirconium-based antiperspirant.

The above aluminium / zirconium salts may have coordinated and/or bound water in various quantities and/or may be present as polymeric species, mixtures or complexes. In particular, zirconium hydroxy salts often represent a range of salts having various amounts of the hydroxy group. Zirconium aluminium chlorohydrate may be particularly preferred.

Antiperspirant complexes based on the above-mentioned astringent aluminium and zirconium salts can be employed, and in particular complexes with chlorohydrate salts. The complex often employs a compound with a carboxylate group, and advantageously this is an amino acid. Examples of suitable amino acids include dl-tryptophan, dl-phenylalanine, dl-valine, dl-methionine and alanine, and preferably glycine which has the formula CH₃CH(NH₂)CO₂H.

It is highly desirable to employ complexes of a combination of aluminium halohydrates and zirconium chlorohydrates together with amino acids such as glycine, which are disclosed in US-A-3792068 (Luedders et al). Certain of those Al/Zr complexes are commonly called ZAG in the literature. ZAG actives generally contain aluminium, zirconium and chloride with an Al/Zr ratio in a range from 2 to 10, especially 2 to 6, an Al/Cl ratio from 2.1 to 0.9 and a variable amount of glycine.

The antiperspirant salt desirably has a mole ratio of metals (Al and/or Zr) to chlorine of from 0.9: to 1.5:1. Some desirable salts have a combined mole ratio of Al + Zr : Cl in the region of 1.3 or 1.35:1 up to 1.5:1, and others a lower mole ratio, such as from 0.9:1 or 1.1:1 up to 1.3:1, e.g. from 1.2:1 to 1.3:1.

The proportion of antiperspirant salt in the invention compositions is normally calculated excluding the weight of any water of hydration but including any complexing agent that may be present, such as glycine.

In addition to water and astringent salt, the disperse phase can contain, if desired, a water-soluble alcohol, which may be monohydric, or polyhydric. Commonly, the weight proportion of alcohol is less than 25% of the disperse phase and often less than 15%. To enhance skin cooling, in some embodiments, the alcohol can be an aliphatic alcohol having a boiling point of below 100°C, such as particularly ethanol.

Preferably, in other embodiments that seek to counteract the astringency of the antiperspirant salt, the alcohol is a water-soluble di or trihydric alcohol that is capable of acting as an humectant, such as glycerol or a PEG oligomer having a molecular weight (weight average) of from 180 to 420. Such an humectant is advantageously present in a weight amount of 0% up to 10% of the emulsion, e.g. at least 0.5%, and n a number of embodiments particularly from 1 to 6%. In such embodiments, any skin-cooling alcohol as described above is present as less than a half, particularly less than a quarter by weight of the total alcohol (by weight) in the emulsion and most preferably is absent.

### Continuous Phase

The continuous phase of the invention emulsions comprises at least one water-immiscible liquid, i.e. an oil, and in many desirable embodiments, a mixture of more than one oil. Herein, the water-immiscible liquids (oils, fluids) have a melting point of 20°C or lower. Particularly, for an antiperspirant composition, the continuous phase commonly represents up to 65 or 70% by weight of the emulsion, and in many desirable embodiments at least 35%. Convenient ranges for the continuous phase are from 35 or 45 to 55 or 60% by weight of such emulsions.

The continuous phase employed in the instant invention often desirably comprises one or more volatile silicone oils. By volatile herein is meant having a measurable vapour pressure at 20 or 25°C. Typically, the vapour pressure of a volatile silicone oil lies in a range from 1 or 10 Pa to 2 kPa at 25°C. Volatile silicone oils can be linear or cyclic siloxanes, usually containing from 3 to 9 silicon atoms, and commonly from 4 to 6 silicon atoms, the silicon atoms being substituted by methyl groups, so that their alternative names are methicones and cyclomethicones. It is especially desirable to employ volatile silicone oils in which at least 80% by weight and particularly at least 90% contain at least 5 silicon atoms, such as cyclopentadimethylsiloxane (D5), cyclohexadimethylsiloxane (D6), dodecamethylpentasiloxane and tetradecamethylhexasiloxane. The cylomethicone oils are especially preferred. Such oils are highly desirable for many consumers because they can evaporate without causing undue skin cooling. The volatile silicone oils often comprise at least 30% by weight of the oil phase and normally not higher than 95% thereof. In a number of desirable compositions their weight proportion in the oil phase is up to 60%, and a preferred range in the oil phase is from 35 to 55% by weight.

The carrier oils can alternatively or additionally comprise one or more non-volatile oils, which can be silicone oils and/or non-silicone oils. Non-volatile silicone oils employed herein preferably contain one or more unsaturated substituents such as phenyl or diphenylethyl in replacement of the corresponding number of methyl substituents in polycyclosiloxanes or more preferably in linear siloxanes, often having 2 or 3 silicon atoms. Such non-volatile oils have a higher refractive index than that of the volatile silicone oils and tend to mask the antiperspirant active when it is deposited on skin. The non-volatile oils can also comprise dimethiconols, which as the name suggests are hydroxyl-terminated. The proportion of non-volatile silicone oils in the oil phase is commonly up to 25% by weight such as from 0.25 to 20% by weight of the phase. In some highly desirable embodiments, the oil phase comprises from 1 to 10% of the non-volatile silicone oil, and in other chosen embodiments, the non-volatile silicone oil provides from 10 to 20% by weight of the oil phase.

The oil phase can alternatively or additionally comprise one or more hydrocarbon fluids, which can be either volatile or non-volatile. Suitable hydrocarbon fluids include liquid aliphatic hydrocarbons such as mineral oils or hydrogenated polyisobutene, desirably selected to exhibit a low viscosity. Further examples of liquid hydrocarbons are polydecene and paraffins and isoparaffins of at least 10 carbon atoms. Hydrocarbon fluids conveniently comprise from 0 to 25% by weight of the oil phase.

In at least some advantageous embodiments, the oil phase comprise liquid aliphatic or aromatic ester oils. Such oils can act as emollients and in addition can affect the sensory attributes of the resultant emulsion. Suitable aliphatic esters contain at least one long chain alkyl group, such as esters derived from C₁ to C₂₀ alkanols esterified with a C₈ to C₂₂ alkanoic acid or C₆ to C₁₀ alkanedioic acid. The alkanol and acid moieties or mixtures thereof are preferably selected such that they each have a melting point of below 20°C. Aliphatic esters include isopropyl myristate, lauryl myristate, isopropyl palmitate, diisopropyl sebacate and diisopropyl adipate. Further and very suitable ester oils include glyceride oils and in particular triglyceride oils derived from glycerol and fatty acids containing at least 6 carbons and especially natural oils.

It is especially desirable to employ aromatic ester oils compared, for example with aliphatic ester oils, in view of their physical properties, such as refractive index. Suitable liquid aromatic esters include fatty alkyl benzoates. Examples of such esters include suitable C₈ to C₁₈ alkyl benzoates or mixtures thereof, including in particular C₁₂ to C₁₅ alkyl benzoates e.g. those available under the trademark Finsolv TN. Such benzoate esters are especially desirable for employment in an aqueous antiperspirant emulsion, and one employing a wax to gel the oil phase, relative to alkyl mono or diesters mentioned above, by virtue of their properties that promote superior aesthetic attributes. An aryl benzoate, such as benzyl benzoate can also be used. Yet other suitable ester oils includes oils in which a short alkylene group of 1 to 3 carbons, optionally substituted by a methyl group, is interposed between benzene and benzoate residues. It is advantageous to select an ester oil having a refractive index of at least 1.47.

The total proportion of ester oils, including both aliphatic and aromatic ester oils (but excluding any fragrance oil) is often from 0 to 50% by weight of the oil phase, is desirably at least 5% by weight and with benefit comprise at least 20% by weight. Their total proportion is in many embodiments desirably from 25% up to 40% of the oil phase. The weight ratio of aromatic ester oil to aliphatic ester oil is often selected in the range of from 1:2 to 20:1, such from 1:1·to 8:1.

Natural ester oils which desirably can be employed herein comprise one or more unsaturated C18 fatty acid glycerides. In many instances, such ester oils comprise one or more triglycerides. The fatty acid residues in the oils can comprise, commonly, from one to three olefinic unsaturated bonds and often one or two. Whilst in many instances the olefinic bonds adopt the trans configuration, in a number of desirable products the bond or bonds adopt the cis configuration. If two or three olefinic unsaturated bonds are present, they can be conjugated. The fatty acid can also be substituted by an hydroxyl group. The natural oils employable herein desirably comprise one or more triglycerides of oleic acid, linoleic acid, linolenic acid or ricinoleic acid. Various isomers of such acids often have common names, including linolenelaidic acid, trans 7-octadecenoic acid, parinaric acid, pinolenic acid punicic acid, petroselenic acid and stearidonic acid. It is especially desirable to employ glycerides derived from oleic acid, linoleic acid or petroselenic acid, or a mixture containing one or more of them.

Natural oils containing one or more of such triglycerides include coriander seed oil for derivatives of petroselinic acid, impatiens balsimina seed oil, parinarium laurinarium kernel fat or sabastiana brasilinensis seed oil for derivatives of cis-parinaric acid, dehydrated castor seed oil, for derivatives of conjugated linoleic acids, borage seed oil and evening primrose oil for derivatives of linoleic and linolenic acids, aquilegia vulgaris oil for columbinic acid and sunflower oil, olive oil or safflower oil for derivatives of oleic acid, often together with linoleic acids. Other suitable oils are obtainable from hemp, which can be processed to derive stearadonic acid derivatives and maize corn oil. An especially convenient natural oil by virtue of its characteristics and availability comprises sunflower oil, ranging from those rich in oleic acid glycerides to those rich in linoleic acid glycerides, rich indicating that its content is higher than that of the other named acid.

The proportion of the natural oil, viz particularly the triglyceride oils of unsaturated fatty acids, if present in the composition, is often selected in the range of from 0.1 to 10% by weight of the oil phase, desirably at least 0.5% by weight and in some embodiments particularly in the range of up to 8% by weight of the phase.

Some preferred embodiments employ a natural ester oil in the oil phase together with a water-soluble humectant, such as those mentioned hereinbefore, in the aqueous phase, and particularly in a weight ratio to each other of from 3:1 to 1:3.

A further class of particularly suitable oils comprises a) non-volatile liquid aliphatic ethers derived from at least one fatty alcohol that desirably contains at least 10 carbon atoms, such as myristyl ether derivatives e.g. PPG-3 myristyl ether or lower alkyl (≤ C₆) ethers of polygylcols (preferably polypropylene glycol and especially 10 to 20 units, such as an ether named as PPG-14 butyl ether in the CTFA. The class of non-volatile ethers additionally comprises b) dialkyl ethers derived from C8 or C10 linear aliphatic alcohols, and particularly dioctyl ether. The invention emulsions herein can alternatively or additionally comprise a dialkyl carbonate derived from C8 or C10 linear aliphatic alcohols, and particularly dioctyl carbonate. It is often convenient for the aliphatic ether or dialkykl carbonate to constitute at least 0%, and especially at least 10%, particularly up to 50% and very desirably up to 30%, %s being by weight of the oil phase.

A further class of oils that can be employed herein comprises water-immiscible aliphatic alcohols, and particularly those having a boiling point of higher than 100°C. These include branched chain alcohols of at least 10 carbon atoms and in many instances up to 30 carbon atoms, particularly 15 to 25, such as isostearyl alcohol, hexyl-decanol and octyl-dodecanol. It will be recognised that octyl-dodecanol is an especially favoured water-immiscible aliphatic alcohol in the instant compositions, because it not only acts as an emollient oil but additionally moisturises skin by the mechanism of occlusion. Other suitable water-immiscible alcohols include intermediate chain length linear alcohols, commonly containing from 9 to 13 carbon atoms, such as decanol or dodecanol. Such alcohols can often constitute at least 0.1% and particularly at least 0.5% by weight of the oil phase, in many compositions being not more than 5% of the phase.

The instant compositions preferably contain a fragrance oil normally at least 10 and often at least 20 fragrance constituents, some of which are sometimes extracted from flora or fauna and others of which are synthesised that are blended together to produce a perfume that is pleasing to the user of the composition. Such a fragrance oil is normally a complex mixture of chemical classes and accordingly, its proportion is excluded from calculations of the proportion of the other constituents of the carrier mixture, including specifically ester, ether and alcohol classes. The fragrance oil commonly will partition between the oil and aqueous phases. The proportion of fragrance oil remaining in the oil phase is usually the major proportion, but will vary depending on the blend of fragrance components and the choice of carrier oils and is accordingly at the discretion of the antiperspirant composition manufacturer. The total weight proportion of fragrance oils in the emulsion is normally selected in the range of from 0 to 4% by weight of the emulsion, often from 0.3% to 2% by weight.

The weight ratio of volatile to non-volatile oils is desirably from 2:1 to 1:2, and particularly from 3:2 to 2:3.

An essential constituent of the oil phase is the hydrocarbon wax comprising the polyethylene having an intermediate low average molecular weight, and especially is selected in the range of from at least 360 daltons up to 460 daltons, or a mixture of said polyethylene waxes. Said wax is present in a proportion high enough to solidify the emulsion. In many desirable embodiments, the weight proportion of the hydrocarbon wax is from 18 to 40% of the oil phase, and especially desirably up to 30%. Expressed in relation to the antiperspirant emulsion, the hydrocarbon wax, including the intermediate weight polyethylene wax, preferably constitutes at least 10.5% by weight and advantageously up to 16% by weight. The hydrocarbon wax can, very suitably, consist predominantly or completely of the intermediate weight polyethylene, such as at least 95% by weight of the wax, and particularly 100%. Said intermediate weight polyethylene is present in the oil phase in a proportion of at least 12.5% by weight of the oil, i.e. at least 1/8th.

Reduction in the total proportion of hydrocarbon wax or/and proportion of intermediate weight polyethylene wax increases the risk of an antiperspirant emulsion stick not being formed at all, i.e. at best being a soft solid which lacks the physical integrity of a firm stick and simply flows under the influence of gravity at ambient temperature unless held within a container.

In addition to said polyethylene, the hydrocarbon wax can additionally comprise a further (supplementary) wax that is itself a hydrocarbon wax such as a petroleum wax, including particularly a paraffin wax, or a mineral wax or ozocerite. Desirably, such further wax, if included, is present in a weight ratio to the polyethylene of less than 1:1. In certain desirable embodiments, the further wax such as the paraffin wax constitutes from 30 to 45% by weight of the hydrocarbon wax. The further wax may alternatively or additionally comprise a lower molecular weight polyethylene wax of average molecular weight of from 260 to 355 daltons.

The further wax advantageously melts at a temperature of at least 60°C up to about 80°C and particularly suitably in the range of from 70 to 80°C. By the selection of such a further wax, it is possible to employ lower processing temperatures, including the temperature at which the fluid stick composition is poured into its dispensing container wax, but by restricting the proportion of the total wax constituted by the further wax, the advantageous properties deriving from the use of the intermediate weight polyethylene can be retained. Lower processing temperatures offer environmental benefits, including energy saving and reduced evaporation.

It is highly desirable to select the total amount of hydrocarbon wax and the proportion of the intermediate molecular weight polyethylene wax such that the resultant stick has a hardness of below 12.5mm needle penetration in the standard penetrometer text specified hereinafter and preferably below 11mm. Such an invention stick can demonstrate a combination of aesthetically desirable properties, including the avoidance of excessive drag on application as well as lower visible marks. In many especially desirable embodiments of the present invention, the resultant stick has a needle penetration of from 7 to 10mm.

### Emulsifier

In order to assist the formation and stability of an emulsion, the invention composition contains a small amount of at least one silicone emulsifier. The emulsifier is very desirably a dimethicone copolyol having an HLB value of from up to 8 and particularly from 3 to 7, such an alkyl dimethicone copolyol.

The proportion of the emulsifier in the composition is often selected in the range of from 0.125% to 1.5% and in many embodiments from 0.15 to 1.0%. In some desirable emulsions, a comparatively low level of emulsifier is present, for example up to 0.5% and in further embodiments a higher level of from 0.5 to 1.5%. In some of the further embodiments, it is preferred to employ from 0.5 to 1.0% of the emulsifier and in others to include slightly more emulsifier of from 1.0 to 1.5% of the emulsion, for example to allow for a fraction of the emulsifier that may be retained within one phase rather than migrating entirely to the interface with the other phase.

### Optional ingredients

The compositions herein can incorporate one or more cosmetic adjuncts conventionally contemplatable for cosmetic solids. Such cosmetic adjuncts can include skin feel improvers, such as talc or finely divided high melting point polyethylene (>110°C), for example in an amount of up to about 6% and often in total in an amount of from 0.5 to 5%; inorganic particulates, preferably finely divided, such as fumed silica, for example in an amount of up to 2%; skin benefit agents such as allantoin, vitamins or lipids, for example in an amount of up to 5%; colours; preservatives such as butylhydroxytoluene, often in an amount of from 0.01 to 0.1%; metal chelates, such as EDTA, for example in an amount of up to 1%; skin cooling agents, such a menthol and menthol derivatives, often in an amount of up to 2%, all of such percentages being by weight of the composition.

A further optional ingredient can comprise a wash-off agent, which can assist a user of the invention composition to wash it off. Such an agent, when employed, often comprises a non-ionic surfactant, such as in an amount of from 0.1 to 2% by weight of the emulsion, and often at least 0.5%. The wash-off agent can be the same as the emulsifier it is preferably a nonionic surfactant such as POE or POP/POE ester or ether (or mixture) having an HLB that is at least 10.

The invention stick compositions can be made in a process comprising in one step making an aqueous phase by blending together the water and water-soluble ingredients, including the antiperspirant astringent salt, at least a fraction of which may be in the form of a pre-formed aqueous solution, in a second step forming a solution of the polyethylene wax in the oil or oil mixture, which normally entails heating the wax and oils to a temperature at which wax particles are no longer visible, commonly in the region of the melting point of the wax, and in a third step mixing the aqueous phase and the oil phase together, and preferably shear mixing the two phases, in the presence of the emulsifier, filling the resultant mixture into a dispenser whilst it is still mobile and thereafter cooling or allowing the composition to cool, initially until the mixture solidifies and then attains ambient temperature.

Steps one and two can be carried out simultaneously or sequentially, though the solution of wax is maintained in a mobile state before step 3. In step 2, the oil is often heated to a temperature in the region of 80 to 85°C, which corresponds within a few degrees to the melting point of the wax. It is often convenient to incorporate ingredients which are not water-soluble, such as water-insoluble optional ingredients and/or the emulsifier in step 2. If desired, the wax or waxes, or a fraction of them, can be heated externally, for example melted, prior to introduction into the oil phase or emulsion.

In a further aspect of the present invention there is provided a stick composition according to the first aspect of the present invention which is contained in solid form in a dispenser. A dispenser for a stick composition often comprises a tubular barrel, having a side wall that surrounds the composition and defines at a first end an opening through which the composition can pass and a second, opposed end within which is fitted a piston, located within the barrel, that is capable of being impelled towards the first end. The means for impelling the piston can comprise a finger or like implement that is pressed by the user against the exposed side of the piston, or a mechanism for imparting axial movement to the piston. Such a mechanism can comprise a threaded spindle that engages with a threaded aperture in the piston, the spindle being mounted on an exposed rotor wheel or fitted with a cog that can be rotated by engagement with a button via a ratchet. The rotor wheel or button is mounted on the barrel adjacent to the second end. Suitable dispensers for firm sticks are described, for example in US 4232977, US4605330, WO09818695, WO09603899, WO09405180, WO09325113, WO09305678, EP1040445, US5997202, US5897263, US5496122, US5275496, US 6598767, US 6299369, or WO 2002/03830.

The present invention includes the non-therapeutic use of the composition according to the present invention described herein in order to inhibit or control perspiration, especially in the armpit. In this aspect, the composition is wiped across the skin to apply it topically, and locally at ambient temperature. The action can be repeated until the user considers that sufficient composition has been deposited, often in the region of 3 to 8 wipes per armpit. The composition is commonly applied shortly after the armpit has been washed or shaved. The composition is thereafter left in place, conventionally, for a period of time commonly between 5 and 24 hours until it is washed off, usually using soap or a conventional shower gel, and water, for example applied using a flannel, loofah, sponge or even fingers. When seeking to inhibit perspiration, the weight of antiperspirant active applied per armpit is often in the range of from 0.15 to 0.5 grams.

Having described the invention and certain preferred embodiments thereof, specific embodiments will now be described more fully by way of example only.

In the Examples and Comparison, the following ingredients were employed:-

**Table 1**

| Ref | INCI name | Trade name / Supplier |
|---|---|---|
| Astringent Active Salt | | |
| AP1 | Aluminium zirconium tetrachlorhydrex GLY | Rezal 36GP, Reheis |
| AP2 | Aluminium zirconium tetrachlorhydrex GLY | AAZG Q5-7167, Summit |
| Structurant | | |
| S1 | Polyethylene Wax | Performalene 400, New Phase technologies |
| S2 | Stearyl alcohol | Lorol C18 Deo, Cognis |
| S3 | Castor Wax | Castorwax MP80, CasChem |
| S4 | Styrene ethylene/ butylene copolymer | Kraton 1650E, Kraton Polymers |
| S5 | Polyethylene wax | Jeenate 3H, Jeen International Corp |
| S6 | C30-45 alkyl methicone and C30-45 olefin | AMSC30, Dow Corning |
| S7 | Paraffin Wax | SP173P, Strahl & Pitsch Inc |
| S8 | Cetearyl behenate | Kesterwax K62, Koster Keuhnen |
| Oils | | |
| O1 | Cyclomethicone | DC245, Dow Corning |
| O2 | C₁₂₋₁₅ Alkyl Benzoate | Finsolv TN, Finetex |
| O3 | PPG-14 Butyl ether | Fluid AP, Amerchol |
| O4 | Neopentyl Glycol Diheptanoate | LexFeel 7, Inolex Chemical Co. |
| O5 | Cetyletherhexanoate | Tegosoft CO, Goldschmidt |
| O6 | Ethylhexylstearate | Tegosoft OS, Goldschmidt |
| O7 | Stearylheptanoate | Tegosoft SH, Goldschmidt |
| O8 | Isocetylstearoyl stearate | Ceraphyl 791, ISP Van Dyk |
| O9 | Octyldodecylstearate | Ceraphyl ODS, ISP Van Dyk |
| O10 | Isocetylneopentanoate | Ceraphyl SLK, ISP Van Dyk |
| O11 | Dimethicone | DC200 (50cSt) Dow Corning |
| O12 | Dimethicone | DC200 (1000cSt) Dow Corning |
| O13 | Helianthus Anuus Oil | Agri Pure 80, Cargill Industrial Oils and Lubricants |
| F | Parfum | various |
| Other | | |
| W1 | Demineralised water | In-house preparation |
| W2 | Glycerin | Pricerine 9091, Uniqema |
| W3 | PEG-8 Distearate | Estol E04DS, Uniqema |
| W4 | Steareth-20 | Brij 700 Uniqema |
| E1 | Cetyl Dimethicone Copolyol | Abil EM90, Goldschmidt |
| T1 | Talc | Suprafino Talc, Luzenac AM |
| T2 | Talc | IMP 1887L, Luzenac America, Inc |
| T3 | Corn Starch | Dry Flo AF, National Starch & Chemical Co |
| T4 | Polyethylene (micronised powder) | Acumist B18, Allied Signal |
| T5 | Hydrophobic silica | HDK H30, Wacker Chemie |
| Alum1 | potassium alum sulphate, 25% w/w solution in demineralised water | Dissolved in house in W1 |
| Alum2 | potassium alum sulphate, 46% w/w solution in demineralised water | Dissolved in house in W1 |

The Examples were made by the following general method. An aqueous solution of the antiperspirant active was made in a first vessel by dissolving it in the demineralised water with heating to a temperature of about 50°C and agitation. The oils were introduced into a second vessel, followed by the emulsifier and the polyethylene wax. The mixture was agitated and heated to a temperature of about 85°C and held at that temperature until the wax particles were no longer visible. The aqueous solution of antiperspirant was then poured slowly into the second vessel whilst the mixture was subjected to shear mixing by a Silverson mixer equipped with a paddle stirrer rotating at about 7500 to 8000rpm and a standard square hole high shear screen. Fragrance, if present, was introduced last. The resultant mixture was poured at the specified temperature, of if not specified at about 80°C, whilst it was still free-flowing, into conventional screw-up stick dispensers (a plastic oval tubular barrel fitted at its lower end with a piston with a central screw threaded aperture which engages a screw threaded spindle that passes through the piston and is mounted on a thumbwheel at the base of the barrel). The filled dispensers were then allowed to cool to ambient temperature (about 22°C).

The Comparison stick was made by a related process in which all the ingredients except the antiperspirant salt were introduced into a vessel equipped with a stirrer and placed on a heater. The contents were then stirred heated to a temperature of about 80 to 85°C until the wax gellant had dissolved. The resultant mobile mixture was allowed to cool to about 75°C, the particulate antiperspirant salt introduced with stirring and the resultant mixture allowed to cooled further to about 60-65°C and then poured into further samples of the same dispenser as for the Example compositions.

The hardness of sticks was measured herein by a standard needle penetration test, using a lab plant PNT penetrometer equipped with a Seta wax needle (weight 2.5 grams in a holder of 47.5 grams) which has a cone angle at the point of the needle specified to be 9°10' ± 15', initially resting on the surface of a freshly cut, flat topped sample, and measuring the depth of penetration after five seconds.

### Example 1 and Comparison.

The Example and Comparison sticks had the compositions and sensory characteristics summarised in Table 2 below. The sensory characteristics were obtained by the following methodology.

A panel of a minimum of 15 trained and experienced female panellists aged between 18 and 55 swiped a stick composition across shaved armpit which had been washed with a soap solution, rinsed with warm water and dried until 300mg of composition had been applied. The sensory attributes, drag and visible deposits, were assessed by each panellist against an anchored scale from 0 to 100, 0 indicating no drag and 100 indicating very considerable drag. The results were averaged.

**Table 2**

| Material | Example 1 | Comparison CA |
|---|---|---|
| | % by weight | |
| Astringent Active Salt | | |
| AP1 | 24.0 | |
| AP2 | | 24.0 |
| Structurant | | |
| S1 | 12.0 | |
| S2 | | 17.5 |
| S3 | | 2.5 |
| Oils | | |
| O1 | 20.4 | 33.3 |
| 02 | 19.0 | |
| 03 | | 17.5 |
| Others | | |
| F | 0.5 | 1.2 |
| W1 | 24.0 | |
| T1 | | 2.0 |
| W3 | | 2.0 |
| E1 | 0.1 | |
| Sensory Characterisation | | |
| Drag (t=0) | 17 | 24 |
| Visible deposits | 8 | 14 |

From Table 2, it can be seen that the Example product exhibited noticeably better drag characteristics (significant at 99% confidence level and noticeably lower visible deposits at the 95% confidence level than did the comparison.

### Examples 2 to 5

In these Examples, further compositions were made in accordance with the invention, as summarised in Table 3 below, employing different amounts of polyethylene wax:-

**Table 3**

| | Ex 2 | Ex3 | Ex4 | Ex5 |
|---|---|---|---|---|
| Material | % by weight | | | |
| S1 | 15 | 10 | 12.5 | 11 |
| O1 | 28 | 25 | 23.5 | 24 |
| O2 | 16 | 24 | 23 | 24 |
| AP1 | 24 | 24 | 24 | 24 |
| W1 | 16 | 16 | 16 | 16 |
| E1 | 0.5 | 0.5 | 0.5 | 0.5 |
| F | 0.5 | 0.5 | 0.5 | 0.5 |
| Total | 100 | 100 | 100 | 100 |

All the sticks summarised in table 3 had acceptable hardness, in a standard needle penetration test, none on average were penetrated by more than 10.8mm.

### Examples 6 to 14

In these Examples, compositions were made as summarised in Table 4 below, employing other non-volatile oils.

**Table 4**

| Material | Ex6 | *Ex7* | *Ex8* | *Ex9* | *Ex10* | *Ex11* | *Ex12* | *Ex13* | *Ex14* |
|---|---|---|---|---|---|---|---|---|---|
| | % by weight | | | | | | | | |
| S1 | 15 | 15 | 15 | 15 | 15 | 15 | 15 | 15 | 15 |
| O1 | 28 | 28 | 28 | 28 | 28 | 28 | 28 | 28 | 28 |
| O2 | 6 | 6 | 6 | 6 | 6 | 6 | 6 | 6 | 6 |
| O4 | 10 | | | | | | | | |
| O5 | | 10 | | | | | | | |
| O6 | | | 10 | | | | | | |
| O7 | | | | 10 | | | | | |
| O8 | | | | | 10 | | | | |
| O9 | | | | | | 10 | | | |
| O10 | | | | | | | 10 | | |
| O11 | | | | | | | | 10 | |
| O12 | | | | | | | | | 10 |
| AP1 | 24 | 24 | 24 | 24 | 24 | 24 | 24 | 24 | 24 |
| W1 | 16 | 16 | 16 | 16 | 16 | 16 | 16 | 16 | 16 |
| E1 | 0.5 | 0.5 | 0.5 | 0.5 | 0.5 | 0.5 | 0.5 | 0.5 | |
| F3 | 0.5 | 0.5 | 0.5 | 0.5 | 0.5 | 0.5 | 0.5 | 0.5 | 0.5 |

### Example 15 to 22

In these Examples, further formulations were made containing one or more optional ingredients, as summarised in Table 5:-

**Table 5**

| | Ex19 | Ex20 | Ex21 | Ex22 |
|---|---|---|---|---|
| Material | % by weight | | | |
| S1 | 10 | | 10 | 12 |
| O1 | 24 | | 24 | 19 |
| O2 | 23 | | 23 | 18 |
| T3 | | | 2 | |
| S4 | | | | 2 |
| T4 | 2 | | | |
| T2 | | 2 | | |
| AP1 | 24 | 24 | 24 | 24 |
| W1 | 16 | 16 | 16 | 16 |
| E1 | 0.5 | 0.5 | 0.5 | 0.5 |
| F | 0.5 | 0.5 | 0.5 | 0.5 |
| Total | 100 | 100 | 100 | 100 |

### Examples 23 to 30

In these Examples, further formulations were made containing a further oil and an humectant, as summarised in Table 6:-

**Table 6**

| | Ex23 | Ex24 | Ex25 | Ex26 | Ex27 | Ex28 | Ex29 | Ex30 |
|---|---|---|---|---|---|---|---|---|
| Material | % by weight | | | | | | | |
| AP1 | 24.00 | 24.00 | 24.00 | 24.00 | 24.00 | 24.00 | 24.00 | 24.00 |
| W1 | 22.00 | 20.00 | 18.00 | 24.00 | 24.00 | 24.00 | 16.00 | 16.00 |
| O1 | 19.90 | 19.90 | 19.90 | 19.90 | 19.90 | 19.90 | 19.90 | 11.00 |
| O2 | 19.00 | 19.00 | 19.00 | 17.00 | 15.00 | 13.00 | 17.00 | 12.00 |
| S1 | 12.00 | 12.00 | 12.00 | 12.00 | 12.00 | 12.00 | 12.00 | |
| F4 | 1.00 | 1.00 | 1.00 | 1.00 | 1.00 | 1.00 | 1.00 | 1.00 |
| E1 | 0.10 | 0.10 | 0.10 | 0.10 | 0.10 | 0.10 | 0.10 | 0.10 |
| W2 | 2.00 | 4.00 | 6.00 | - | - | - | 8.00 | 8.00 |
| O13 | - | - | - | 2.00 | 4.00 | 6.00 | 2.00 | 8.00 |
| F | | | | | | | | 1.00 |
| Total | 100 | 100 | 100 | 100 | 100 | 100 | 100 | 100 |

Stability tests indicated that all the formulations summarised in Table 6 were stable at both 50°C and 60°C. The sticks all had acceptable hardness, but on average were similar to the sticks summarised in Table 3, having a needle penetration in the standard test though that of Example 25 was slightly softer at 11.7mm penetration.

### Examples 31 to 36

In these Examples, further formulations were made containing a wash-off agent as summarised in Table 7:-

**Table 7**

| | Ex31 | Ex32 | Ex33 I | Ex34 | Ex35 | Ex36 |
|---|---|---|---|---|---|---|
| Material | % by weight | | | | | |
| AP1 | 24.00 | 24.00 | 24.00 | 24.00 | 24.00 | 24.00 |
| W1 | 20.00 | 20.00 | 20.00 | 20.00 | 20.00 | 20.00 |
| O1 | 19.20 | 19.30 | 19.40 | 19.35 | 19.05 | 18.85 |
| O2 | 18.35 | 18.45 | 18.55 | 18.50 | 18.20 | 18.00 |
| S1 | 12.00 | 12.00 | 12.00 | 12.00 | 12.00 | 12.00 |
| E1 | 0.70 | 0.50 | 0.30 | 0.40 | 1.00 | 1.40 |
| W2 | 4.00 | 4.00 | 4.00 | 4.00 | 4.00 | 4.00 |
| F | 1.00 | 1.00 | 1.00 | 1.00 | 1.00 | 1.00 |
| W4 | 0.75 | 0.75 | 0.75 | 0.75 | 0.75 | 0.75 |
| Total | 100 | 100 | 100 | 100 | 100 | 100 |

### Comparisons CB and CC

In these comparisons, further formulations were made containing a polyethylene structurant with a lower average molecular weight by the same general method as for Example 1, as summarised in Table 8:-

**Table 8**

| | CB | CC |
|---|---|---|
| Material | % by weight | |
| S5 | 12.00 | 15.00 |
| AP1 | 24.00 | 24.00 |
| W1 | 24.00 | 24.00 |
| O1 | 19.90 | 18.35 |
| O2 | 19.00 | 17.55 |
| E1 | 0.10 | 0.10 |
| F | 1.00 | 1.00 |
| Total | 100.00 | 100.00 |
| Hardness | 14mm | 11.9mm |

Formulation CB, employing a polyethylene wax having a lower molecular weight than the intermediate molecular weight polyethylene wax according to the instant invention, suffered from inferior hardness, having a penetration of 14mm compared with a hardness commonly below 10mm or 11mm when employing a polyethylene wax according to the invention. The hardness was improved by increasing the proportion of the wax structurant, as in comparison CC, but that resulted in a noticeably worse wash-off when the product was applied to skin.

### Example 37 and Comparisons CD and CE

In comparisons CD and CE, comparative examples were prepared by the general method for Example 1 (as used also for Example 37), but substituting potassium alum (KAl (SO₄)₂.12H₂O) for an aluminium zirconium antiperspirant active salt. The formulations are summarised in Table 9 below.

Some of the sticks were stored at ambient temperature before being tested and others were subjected to a cycle of freezing and thawing to mimic the effect of temperature changes to which cosmetic formulations are often subjected prior to them being used by the consumer.

The sticks were tested to identify the extent to which they left visible white deposits in the following test. Each stick was wiped five times at a slow constant speed in the same direction across a black cloth stretched taut on a base. The resultant cloth was then compared by eye by a panel in a scale of 0 to 10 anchored at the extremes by two references that had been tested in the same way. The scale value 0 was the cloth itself, and the scale value of 10 was obtained by using a white, wax-structured stick having the formulation of comparison CA summarised in Table 2 above which was known to leave moderate visible deposits.

**Table 9**

| | CD | CE | Ex37 |
|---|---|---|---|
| Material | % by weight | | |
| S1 | 12.00 | 12.00 | 12.00 |
| O1 | 19.75 | 19.75 | 19.75 |
| O2 | 19.00 | 19.00 | 19.00 |
| E1 | 0.25 | 0.25 | 0.25 |
| Alum1 | 12.00 | | |
| Alum2 | | 22.08 | |
| AP1 | | | 24.00 |
| W1 | 36.00 | | 24.00 |
| F | 1.00 | 1.00 | 1.00 |
| Total | 100 | 100 | 100 |

On visual inspection, both formulations CD and CE leaked a visible liquid when rubbed gently on skin and felt gritty, which was indicative of solid particles being formed in the formulation during the cooling and/or storage of the formulations overnight before application. The Example formulation suffered from neither of these sensory or visual negatives.

In the tests, Comparison CE was both visibly worse than the product defining 10 on the scale, whereas that for CD was better, but still at about 7 on the scale and Example 31 had an average score of 2.83 (standard deviation of 0.8), showing a comparatively low visible deposits. These comparisons show clearly that the sticks made containing the alum deodorant, stick structured with polyethylene did not demonstrate low visible deposits, even though they employed a polyethylene wax and suffered other sensory negatives.

### Comparison CF

In this comparison, a stick was made in accordance with the disclosure of EP1280502 in which an antiperspirant emulsion stick structured with an ester wax had been shown to demonstrate comparatively low visible deposits. Its formulation is summarised in Table 10 below.

**Table 10**

| | CF |
|---|---|
| Material | % by weight |
| S8 | 12.5 |
| O1 | 12.6 |
| O2 | 8.4 |
| AP1 | 23.2 |
| E1 | 1.0 |
| W1 | 34.8 |
| W2 | 4.5 |
| T4 | 2.0 |
| F | 1.0 |

The visible deposits of comparison formulation CF was compared with that for Example 31 under the same conditions. The average score from the 10 panellists for CF was 7.17 with a standard deviation of 0.72. The score for Example 31 was, as indicated above, 2.83, showing beyond doubt that the invention stick that had been structured with intermediate molecular weight polyethylene wax demonstrated significantly lower visible deposits than the one structured with the ester wax.

### Example 38

In this Example, the composition of Example 1 was repeated, but substituting 6% of wax S5, a polyethylene wax of lower molecular weight for 6% of the intermediate molecular weight polyethylene wax, S2. The Example 38 composition could be cooled to a temperature approximately 7°C lower than that of Example 1 and retain sufficient fluidity for it to be poured into dispensing canisters.

### Examples 39 to 42

These Examples were made employing the general method employed for Example 1,using the ingredients specified in Table 11 below. It was observed that all of them were able to be poured into the stick canisters at a temperature of below 80°C, whilst at the same time retaining a hardness of below 10mm penetration in the standard penetrometer test.

**Table 11**

| Example | 39 | 40 | 41 | 42 |
|---|---|---|---|---|
| Material | % by weight | | | |
| S1 | 6.00 | 8.00 | 9.00 | 8.00 |
| S6 | 6.00 | | | |
| S7 | | 6.00 | 5.00 | 6.00 |
| O1 | 19.90 | 22.65 | 18.70 | 18.10 |
| O2 | 19.00 | 15.10 | 17.60 | 17.25 |
| E1 | 0.1 | 0.1 | 0.5 | 0.7 |
| AP1 | 24.00 | 24.00 | 24.00 | 24.00 |
| W1 | 20.00 | 20.00 | 24.00 | 24.00 |
| W2 | 4.00 | 4.00 | 4.00 | 4.00 |
| F | 1.00 | 1.00 | 1.00 | 1.00 |
| Total | 100 | 100 | 100 | 100 |

### Examples 43 to 48

These examples were made by the general method employed for the preparation of Examples 40 to 42.

| Example | 43 | 44 | 45 | 46 | 47 | 48 |
|---|---|---|---|---|---|---|
| Material | % by weight | | | | | |
| S1 | 8 | 8 | 8 | 12 | 10 | 12 |
| S7 | 6 | 6 | 6 | 0 | 5 | 0 |
| O1 | 18.7 | 18.7 | 18 | 19.7 | 21.3 | 19 |
| O2 | 17.85 | 17.6 | 17.6 | 18.6 | 14 | 18.6 |
| E1 | 0.25 | 0.5 | 1.2 | 0.5 | 0.5 | 1.2 |
| AP1 | 24 | 24 | 24 | 24 | 24 | 24 |
| W1 | 20 | 24 | 24 | 24 | 24 | 24 |
| W2 | 4 | 0 | 0 | 0 | 0 | 0 |
| F | 1.2 | 1.2 | 1.2 | 1.2 | 1.2 | 1.2 |
| Hardness mm | 11.7 | 12.4 | 11.8 | 8.3 | 8.1 | 9.6 |
| Visible deposits | 2.7 | 2.6 | 3.6 | 2.7 | 5 | 2.6 |

The visible deposits figure quoted for these Examples was measured in the same way as for Example 37, but is an average of 12 panellists after removal of the highest and the lowest score.

All of these Examples 43 to 48 exhibited excellent glide.

## Claims

1. A solid astringent stick composition in the form of an water in oil- emulsion containing an antiperspirant salt and comprising:-
from 34% to 70% by weight of a dispersed aqueous phase containing from 15 to 30% by weight of a water-soluble astringent aluminium-zirconium antiperspirant salt - from 29% to 65% by weight of an oil phase comprising at least one oil and a hydrocarbon wax that solidifies the oil, the hydrocarbon wax comprising at least 1 part w/w of polyethylene of intermediate molecular weight being a weight average molecular weight of from 360 to 460 daltons per 8 parts of oil phase and from 0.125% to 2.0% by weight of a silicone emulsifier, percentages being by weight of the composition.

2. A composition according to claim 1 which contains from 18 to 25% by weight of said water-soluble antiperspirant salt.

3. A composition according to either preceding claim in which the aqueous phase contains said water-soluble antiperspirant salt at a concentration in said phase of from 35 to 55% by weight, and preferably from 45 to 52.5% by weight.

4. A composition according to claim 2 or 3 in which the aqueous phase constitutes from 40 to 55% by weight of the emulsion.

5. A composition according to any preceding claim in which said water-soluble antiperspirant salt is an aluminium zirconium chlorohydrate, optionally complexed.

6. A composition according to claim 6 in which said water-soluble antiperspirant salt is a aluminium zirconium chlorohydrate glycine complex.

7. A composition according to any preceding claim which contains up to 10% by weight, preferably 1 to 6% by weight of a water-soluble polyhydric alcohol.

8. A composition according to claim 1 in which the water-soluble polyhydric alcohol is glycerol.

9. A composition according to any preceding claim in which the oil phase contains at least 15%, by weight based on the oil phase, of the intermediate molecular weight polyethylene.

10. A composition according to any preceding claim in which the oil phase contains from 18 to 28% by weight based on the oil phase, of the intermediate molecular weight polyethylene.

11. A composition according to any preceding claim in which the hydrocarbon wax consists of the intermediate molecular weight polyethylene, either by itself or together with a paraffin wax or lower molecular weight polyethylene in a weight ratio to the polyethylene of up to 1:1.

12. A composition according to any preceding claim in which at least 95% by weight of the hydrocarbon wax consists of the intermediate molecular weight polyethylene.

13. A composition according to claim 11 in which the hydrocarbon wax consists of from 55 to 70% by weight of the intermediate molecular weight polyethylene and the balance of from 30 to 45% of the paraffin wax or lower molecular weight polyethylene.

14. A composition according to any preceding claim in which the emulsion contains from 10.5 to 16% by weight of the hydrocarbon wax.

15. A composition according to any preceding claim in which the oil phase comprises a volatile silicone oil and optionally a non-volatile oil.

16. A composition according to claim 15 in which the volatile silicone oil represents from 30 to 60% by weight of the oil phase.

17. A composition according to claim 15 or 16 in which the oil phase comprises an aromatic ester oil, and preferably an oil having a refractive index of from 1.49 to 1.57.

18. A composition according to claim 17 in which said ester oil is an alkyl benzoate.

19. A composition according to claim 17 or 18 in which the aromatic ester oil represents from 30 to 60% by weight of the oil phase.

20. A composition according to any of claims 15 to 19 in which the aromatic ester oil is present in a weight ratio to the volatile silicone oil of from 2:1 to 1:2.

21. A composition according to any preceding claim in which the emulsifier comprises a dimethicone copolymer, and particularly a polyoxyalkylene modified dimethylpolysiloxane.

22. A composition according to claim 21 in which the emulsifier is present at a concentration of from 0.075 to 0.75% of the composition.

23. A composition according to claim 21 in which the emulsifier is present at a concentration of from 0.5 to 1.5% of the composition.

24. A process for the manufacture of a composition according to any preceding claim in which the hydrocarbon wax comprising polyethylene having a weight average molecular weight of from 360 to 460 daltons is dissolved in an oil to form an oil phase, an aqueous solution of an antiperspirant salt dissolved in water is dispersed in the oil phase in the presence of an emulsifying agent and the oil phase is caused or permitted to solidify.

25. A non-therapeutic method of locally inhibiting or controlling perspiration in which a stick composition according to any one of claims 1 to 23 is wiped across human skin, preferably in the axilla.

## Patentansprüche

1. Feste, adstringierende Stiftzusammensetzung in der Form einer Wasser-in-Öl-Emulsion, die ein transpirationshemmendes Salz enthält und umfasst:
34 bis 70 Gewichts-% einer dispergierten wässrigen Phase, die 15 bis 30 Gewichts-% eines wasserlöslichen adstringierenden transpirationshemmenden Aluminium-Zirkonium-Salzes enthält, 29 bis 65 Gewichts-% einer Ölphase, die wenigstens ein Öl und ein Kohlenwasserstoffwachs, welches das Öl verfestigt, umfasst, wobei das Kohlenwasserstoffwachs wenigstens 1 Teil G/G Polyethylen mit mittlerem Molekulargewicht, das ein Molekulargewicht von 360 bis 460 Dalton ist, pro 8 Teile Ölphase umfasst, und 0,125 bis 2,0 Gewichts-% eines Silikon-Emulgators, wobei Prozentangaben Gewichts-% der Zusammensetzung sind.

2. Zusammensetzung nach Anspruch 1, die 18 bis 25 Gewichts-% des wasserlöslichen transpirationshemmenden Salzes enthält.

3. Zusammensetzung nach irgendeinem vorangehenden Anspruch, wobei die wässrige Phase das wasserlösliche transpirationshemmende Salz in einer Konzentration in der Phase von 35 bis 55 Gewichts-% und vorzugsweise von 45 bis 52,5 Gewichts-% enthält.

4. Zusammensetzung nach Anspruch 2 oder 3, wobei die wässrige Phase 40 bis 55 Gewichts-% der Emulsion ausmacht.

5. Zusammensetzung nach irgendeinem vorangehenden Anspruch, wobei das wasserlösliche transpirationshemmende Salz ein Aluminium-Zirkonium-Chlorhydrat, das gegebenenfalls komplexiert ist, ist.

6. Zusammensetzung nach Anspruch 6, wobei das wasserlösliche transpirationshemmende Salz ein Aluminium-Zirkonium-Chlorhydrat-Glycin-Komplex ist.

7. Zusammensetzung nach irgendeinem vorangehenden Anspruch, die bis zu 10 Gewichts-%, vorzugsweise 1 bis 6 Gewichts-%, eines wasserlöslichen mehrwertigen Alkohols enthält.

8. Zusammensetzung nach Anspruch 8, in der der wasserlösliche mehrwertige Alkohol Glycerin ist.

9. Zusammensetzung nach irgendeinem vorangehenden Anspruch, wobei die Ölphase wenigstens 15 Gewichts-%, bezogen auf die Ölphase, des Polyethylens mit mittlerem Molekulargewicht enthält.

10. Zusammensetzung nach irgendeinem vorangehenden Anspruch, wobei die Ölphase 18 bis 28 Gewichts-%, bezogen auf die Ölphase, des Polyethylens mit mittlerem Molekulargewicht enthält.

11. Zusammensetzung nach irgendeinem vorangehenden Anspruch, wobei das Kohlenwasserstoffwachs aus dem Polyethylen mit mittlerem Molekulargewicht besteht, und zwar entweder selbst oder zusammen mit einem Paraffinwachs oder Polyethylenglykol mit niedrigerem Molekulargewicht in einem Gewichtsverhältnis zu dem Polyethylen von bis zu 1:1.

12. Zusammensetzung nach irgendeinem vorangehenden Anspruch, wobei wenigstens 95 Gewichts-% des Kohlenwasserstoffwachses aus dem Polyethylen mit mittlerem Molekulargewicht bestehen.

13. Zusammensetzung nach Anspruch 11, wobei das Kohlenwasserstoffwachs aus 55 bis 70 Gewichts-% des Polyethylens mit mittlerem Molekulargewicht und als Rest aus 30 bis 45 % des Paraffinwachses oder Polyethylens mit niedrigerem Molekulargewicht besteht.

14. Zusammensetzung nach irgendeinem vorangehenden Anspruch, wobei die Emulsion 10,5 bis 16 Gewichts-% des Kohlenwasserstoffwachses enthält.

15. Zusammensetzung nach irgendeinem vorangehenden Anspruch, wobei die Ölphase ein flüchtiges Silikonöl und gegebenenfalls ein nicht-flüchtiges Öl umfasst.

16. Zusammensetzung nach Anspruch 15, wobei das flüchtige Silikonöl 30 bis 60 Gewichts-% der Ölphase darstellt.

17. Zusammensetzung nach Anspruch 15 oder 16, wobei die Ölphase ein aromatisches Esteröl und vorzugsweise ein Öl, das einen Brechungsindex von 1,49 bis 1,57 hat, umfasst.

18. Zusammensetzung nach Anspruch 17, wobei das Esteröl ein Alkylbenzoat ist.

19. Zusammensetzung nach Anspruch 17 oder 18, wobei das aromatische Esteröl 30 bis 60 Gewichts-% der Ölphase darstellt.

20. Zusammensetzung nach irgendeinem der Ansprüche 15 bis 19, wobei das aromatische Esteröl in einem Gewichtsverhältnis zu dem flüchtigen Silikonöl von 2:1 bis 1:2 vorliegt.

21. Zusammensetzung nach irgendeinem vorangehenden Anspruch, wobei der Emulgator ein Dimethicon-Copolymer und insbesondere ein Polyoxyalkylen-modifiziertes Dimethylpolysiloxan umfasst.

22. Zusammensetzung nach Anspruch 21, wobei der Emulgator in einer Konzentration von 0,075 bis 0,75 % der Zusammensetzung vorliegt.

23. Zusammensetzung nach Anspruch 21, wobei der Emulgator in einer Konzentration von 0,5 bis 1,5 % der Zusammensetzung vorliegt.

24. Verfahren für die Herstellung einer Zusammensetzung nach irgendeinem vorangehenden Anspruch, wobei das Kohlenwasserstoffwachs, das Polyethylen mit einem gewichtsmittleren Molekulargewicht von 360 bis 460 Dalton umfasst, in einem Öl unter Bildung einer Ölphase gelöst wird, eine wässrige Lösung eines transpirationshemmenden Salzes, gelöst in Wasser, in der Ölphase in Gegenwart eines Emulgators dispergiert wird und bewirkt oder zugelassen wird, dass die Ölphase sich verfestigt.

25. Nicht-therapeutisches Verfahren zur lokalen Inhibierung oder Kontrolle des Schwitzens, wobei eine Stiftzusammensetzung nach irgendeinem der Ansprüche 1 bis 23 über menschliche Haut, vorzugsweise in der Achselhöhle, gestrichen wird.

## Revendications

1. Composition solide astringente en stick sous la forme d'une émulsion eau dans l'huile contenant un sel antitranspirant et comprenant :
de 34 % à 70 % en poids d'une phase aqueuse dispersée contenant de 15 % à 30 % en poids d'un sel antitranspirant d'aluminium-zirconium astringent et soluble dans l'eau, de 29 % à 65 % en poids d'une phase huileuse comprenant au moins une huile et une cire hydrocarbonée qui solidifie l'huile, la cire hydrocarbonée comprenant au moins 1 partie m/m de polyéthylène d'un poids moléculaire intermédiaire compris dans la plage allant de 360 à 460 daltons pour 8 parties de phase huileuse et de 0,125 % à 2,0 % en poids d'un émulsifiant siliconé, les pourcentages étant sur la base du poids de la composition.

2. Composition selon la revendication 1, qui comprend de 18 % à 25 % en poids dudit sel antitranspirant soluble dans l'eau.

3. Composition selon l'une quelconque des revendications précédentes, dans laquelle la phase aqueuse comprend ledit sel antitranspirant soluble dans l'eau à une concentration dans ladite phase comprise dans la plage allant de 35 % à 55 % en poids, et de préférence de 45 % à 52,5 % en poids.

4. Composition selon la revendication 2 ou 3, dans laquelle la phase aqueuse représente de 40 % à 55 % en poids de l'émulsion.

5. Composition selon l'une quelconque des revendications précédentes, dans laquelle ledit sel antitranspirant soluble dans l'eau est un chlorhydrate d'aluminium-zirconium, facultativement complexé.

6. Composition selon la revendication 5, dans laquelle ledit sel antitranspirant soluble dans l'eau est un complexe de glycine et de chlorhydrate d'aluminium-zirconium.

7. Composition selon l'une quelconque des revendications précédentes, qui comprend jusqu'à 10 % en poids, de préférence de 1 à 6 % en poids d'un polyol soluble dans l'eau.

8. Composition selon la revendication 1, dans laquelle le polyol soluble dans l'eau est le glycérol.

9. Composition selon l'une quelconque des revendications précédentes, dans laquelle la phase huileuse comprend au moins 15 % en poids, sur la base de la phase huileuse, du polyéthylène de poids moléculaire intermédiaire.

10. Composition selon l'une quelconque des revendications précédentes, dans laquelle la phase huile comprend de 18 % à 28 % en poids, sur la base de la phase huileuse, du polyéthylène de poids moléculaire intermédiaire.

11. Composition selon l'une quelconque des revendications précédentes, dans laquelle la cire hydrocarbonée se compose du polyéthylène de poids moléculaire intermédiaire, seul ou avec une cire de paraffine ou un polyéthylène de poids moléculaire moins élevé selon un rapport en poids par rapport au polyéthylène allant jusqu'à 1/1.

12. Composition selon l'une quelconque des revendications précédentes, dans laquelle au moins 95 % en poids de la cire hydrocarbonée se compose du polyéthylène de poids moléculaire intermédiaire.

13. Composition selon la revendication 11, dans laquelle la cire hydrocarbonée se compose de 55 % à 70 % en poids du polyéthylène de poids moléculaire intermédiaire et le reste, de 30 % à 45 %, de la cire de paraffine ou d'un polyéthylène de poids moléculaire moins élevé.

14. Composition selon l'une quelconque des revendications précédentes, dans laquelle l'émulsion comprend de 10,5 % à 16 % en poids de la cire hydrocarbonée.

15. Composition selon l'une quelconque des revendications précédentes, dans laquelle la phase huileuse comprend une huile de silicone volatile et facultativement une huile non volatile.

16. Composition selon la revendication 15, dans laquelle l'huile de silicone volatile représente de 30 % à 60 % en poids de la phase huileuse.

17. Composition selon la revendication 15 ou 16, dans laquelle la phase huileuse comprend une huile d'ester aromatique, et de préférence une huile possédant un indice de réfraction compris dans la plage allant de 1,49 à 1,57.

18. Composition selon la revendication 17, dans laquelle ladite huile d'ester est un benzoate d'alkyle.

19. Composition selon la revendication 17 ou 18, dans laquelle l'huile d'ester aromatique représente de 30 % à 60 % en poids de la phase huileuse.

20. Composition selon l'une quelconque des revendications 15 à 19, dans laquelle l'huile d'ester aromatique est présente selon un rapport en poids par rapport à l'huile de silicone volatile compris dans la plage allant de 2/1 à 1/2.

21. Composition selon l'une quelconque des revendications précédentes, dans laquelle l'émulsifiant comprend un copolymère de diméthicone, et en particulier un diméthylpolysiloxane modifié par un polyoxyalkylène.

22. Composition selon la revendication 21, dans laquelle l'émulsifiant est présent à une concentration de 0,075 % à 0,75 % de la composition.

23. Composition selon la revendication 21, dans laquelle l'émulsifiant est présent à une concentration de 0,5 % à 1,5 % de la composition.

24. Procédé de fabrication d'une composition selon l'une quelconque des revendications précédentes, dans lequel la cire hydrocarbonée comprenant du polyéthylène ayant un poids moléculaire moyen de 360 à 460 daltons est dissoute dans une huile pour former une phase huileuse, une solution aqueuse d'un sel antitranspirant dissous dans de l'eau est dispersée dans la phase huileuse en présence d'un agent émulsifiant et la solidification de la phase huileuse est provoquée ou autorisée.

25. Procédé non thérapeutique d'inhibition ou de contrôle de la transpiration de manière locale, dans lequel une composition en stick selon l'une quelconque des revendications 1 à 23 est appliquée sur la peau d'un être humain, de préférence au niveau de l'aisselle.
